# FASCICULE DE BREVET EUROPEEN

(11) **EP 2 813 258 B1**
(45) Date de publication et mention de la délivrance du brevet: **16.09.2015**
(21) Numéro de dépôt: 13193370.7
(22) Date de dépôt: 18.11.2013
(51) Int. Cl.: A61N 1/05

(54) **Microsonde implantable de détection/stimulation incorporant un agent anti-inflammatoire**
Implantierbare Detektions-/Stimulations-Mikrosonde, die eine entzündungshemmende Substanz enthält
Implantable detection/stimulation microprobe including an anti-inflammatory agent

(30) Priorité: 11.06.2013 FR 1355385
(43) Date de publication de la demande: 17.12.2014
(73) Titulaire: Sorin CRM SAS, 92140 Clamart Cedex (FR)
(72) Inventeur: D'hiver, Philippe, 92320 CHATILLON (FR)
(74) Mandataire: Dupuis-Latour, Dominique

(56) Documents cités:
- EP-A1- 2 581 107
- WO-A1-01/23034
- WO-A1-2008/013908
- US-A- 5 496 360

## Description

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation et/ou de défibrillation en cas de trouble du rythme détecté par le dispositif.

Elle concerne plus précisément les sondes de stimulation cardiaque destinées à être implantées dans le réseau coronarien du coeur pour permettre la stimulation d'une cavité gauche, ventricule ou oreillette.

Une tendance des développements récents en matière de sondes de stimulation du ventricule gauche est la réduction du diamètre de la partie implantable dans le réseau coronaire. La taille du corps de sonde est en effet un facteur déterminant en ce qui concerne l'aptitude au guidage dans le réseau coronaire veineux, pour pouvoir y sélectionner des sites de stimulation particuliers situés dans certaines veines collatérales.

Le EP 2 581 107 A1 (Sorin CRM SAS) décrit une telle sonde dont la partie active distale présente un diamètre de l'ordre de 0,5 à 2 French (0,17 à 0,66 mm). Elle est formée d'un câble de coeur électriquement conducteur constitué d'un toron de microcâbles eux-mêmes formés de brins composites toronnés ensemble, avec une couche d'isolement en polymère entourant le câble de coeur. La couche d'isolement est dénudée ponctuellement de façon à mettre à nu le câble de coeur en un ou plusieurs points constituant ensemble un réseau d'électrodes reliées en série.

Le très faible diamètre de cette "microsonde" permet d'exploiter toute la longueur de la veine et de canuler des veines de très faible diamètre, non exploitées à ce jour du fait de la taille excessive des sondes coronaires conventionnelles. Il devient de ce fait possible de traiter de nouvelles régions difficilement atteignables et ainsi d'utiliser de façon optimale toutes les veines présentes dans la zone basale, en particulier pour éviter le risque de stimulation phrénique qui augmente généralement lorsque la sonde est trop distale. Avec une telle microsonde, il est même possible de traverser des anastomoses (passages présents à l'extrémité de certaines veines vers une autre veine) en faisant progresser la microsonde dans une première veine (veine "aller") puis par l'anastomose vers une deuxième veine (veine "retour") en remontant celle-ci, pour permettre en particulier une stimulation du ventricule gauche à partir de deux régions distinctes et distantes.

Par ailleurs, la multiplication des points de stimulation dans une zone profonde du réseau coronarien permet (contrairement aux sondes traditionnelles) de stimuler simultanément plusieurs zones de l'épicarde dans la région de stimulation, en améliorant de ce fait les chances d'une resynchronisation optimale du myocarde.

Enfin, la structure de cette microsonde lui procure une grande robustesse qui en garantit la biostabilité à long terme.

L'invention concerne plus particulièrement le phénomène d'inflammation du tissu biologique avec lequel la sonde est en contact mécanique : ce contact exerce une pression et s'accompagne quelquefois de petits mouvements, et ces actions mécaniques ont pour conséquence une inflammation du tissu, qui peut durer quelques semaines.

Il s'agit d'ailleurs d'un phénomène rencontré avec tous les types de sondes, qu'elles soient placées dans des veines coronaires ou dans des cavités cardiaques.

Sur le plan du fonctionnement du dispositif, cette inflammation a pour principale conséquence d'augmenter le seuil de stimulation (seuil de capture) et donc de nécessiter une augmentation de l'énergie des impulsions délivrées, ainsi qu'une réévaluation à intervalles réguliers du seuil de capture de manière à adapter le niveau de l'énergie délivrée aux variations de ce seuil en fonction du degré d'inflammation.

En résumé, l'inflammation induit une surconsommation énergétique du dispositif, ainsi qu'un risque de perte de capture.

La technique conventionnelle pour minimiser ce phénomène d'inflammation consiste à incorporer aux sondes des pièces moulées en silicone chargé d'un agent anti-inflammatoire, généralement un stéroïde, par exemple un glucocorticoïde tel que la dexaméthasone phosphate sodique (ci-après DSP). Ces pièces moulées sont disposées notamment au niveau des électrodes, pour pallier les conséquences de l'inflammation sur l'élévation du seuil de capture, l'agent anti-inflammatoire étant progressivement relargué par diffusion dans et hors du silicone, après implantation de la sonde.

Le US 5 496 360 A est un exemple de sonde conçue selon cette technique, avec une électrode d'extrémité creuse comprenant une chambre interne contentant une matrice polymère imprégnée d'un agent tel que la DSP. La chambre communique avec l'extérieur par un canal axial étroit permettant à la DSP de diffuser lentement autour de l'électrode par effet de "pompe osmotique" avec les fluides environnants.

Cette technique est toutefois complexe et couteuse à mettre en oeuvre du fait des multiples étapes de processus qu'elle implique : tamisage du stéroïde pour contrôler la taille des grains, préparation du mélange de pâte silicone et de stéroïde, moulage et ébavurage, contrôle visuel des pièces moulées, montage manuel sonde par sonde des pièces de silicone chargé de stéroïde.

En tout état de cause, cette technique est en pratique difficilement envisageable pour des microsondes de diamètre inférieur à 0,5 mm, dont la taille extrêmement fine rendrait inadaptée la mise en place de pièces rapportées en silicone chargé d'un stéroïde, ou l'usinage de pièces creuses comme dans le US 5 496 360 A précité.

Tel est le problème que se propose de résoudre l'invention.

Le point de départ de l'invention est la constatation que, dans une microsonde telle que décrite plus haut, les microcâbles du toron constituant le câble de coeur de la microsonde n'occupent en fait qu'une partie du volume intérieur disponible sous la couche d'isolement, en pratique environ la moitié de ce volume.

L'idée de base de l'invention est d'utiliser cet espace disponible pour y incorporer un agent pharmacologiquement actif, en particulier un anti-inflammatoire, qui sera relargué progressivement dans le milieu environnant au niveau des électrodes : ces dernières sont, précisément, constituées par des zones dénudées de la couche d'isolement et permettent de ce fait une communication entre le volume intérieur de la microsonde et l'environnement extérieur.

Plus précisément, l'invention propose une sonde du type général divulgué par le EP 2 581 107 A1 précité, c'est-à-dire comportant une partie distale active dépourvue de lumière centrale et formée par : un câble électriquement conducteur formé par un toron de microcâbles, chacun des microcâbles étant formé d'un toron de brins métalliques individuels ; une couche d'isolement en matériau électriquement isolant, gainant le câble ; au moins une zone dénudée ménagée dans la couche d'isolement de manière à former une électrode correspondante de la microsonde ; et un agent pharmacologiquement actif, destiné à être progressivement libéré dans l'environnement de la microsonde après implantation de celle-ci.

De façon caractéristique de l'invention, l'agent pharmacologiquement actif est présent sous forme d'un matériau soluble, et l'espace interstitiel, délimité par la paroi interne de la couche d'isolement, subsistant entre les brins de chaque microcâble, est rempli par l'agent pharmacologiquement actif.

L'agent pharmacologiquement actif peut être en particulier un agent anti-inflammatoire, notamment un glucocorticoïde tel que la dexaméthasone phosphate sodique ou bien un anti-inflammatoire non stéroïdien.

Dans une forme de réalisation avantageuse, l'extrémité distale de la microsonde comprend un prolongement de la couche d'isolement au-delà de la terminaison dudit câble, ce prolongement définissant un espace vide formant réservoir additionnel pour l'agent pharmacologiquement actif, ce réservoir étant obturé à son extrémité distale par un élément atraumatique.

L'invention a également pour objet un procédé spécialement adapté à la fabrication d'une sonde telle que ci-dessus.

Ce procédé est caractérisé par les étapes suivantes :
a) obtention d'une microsonde comportant une partie distale active formée par : un câble électriquement conducteur formé par un toron de microcâbles dont chacun est formé d'un toron de brins métalliques individuels, une couche d'isolement en matériau électriquement isolant gainant le câble, et au moins une zone dénudée ménagée dans la couche d'isolement de manière à former une électrode correspondante de la microsonde ;
b) immersion de la microsonde obtenue à l'étape a) dans un bain d'une solution d'un agent pharmacologiquement actif ;
c) placement de la microsonde et du bain de l'étape b) dans une enceinte étanche et mise sous vide de l'enceinte, de manière à évacuer l'air subsistant dans l'espace interstitiel, délimité par la paroi interne de la couche d'isolement, subsistant entre les brins de chaque microcâble de la microsonde ;
d) rétablissement d'une pression, ou établissement d'une surpression, dans l'enceinte, de manière à induire la pénétration de l'agent pharmacologiquement actif dans ledit espace interstitiel ; et
e) extraction de la microsonde hors du bain et séchage de la microsonde, de manière à extraire la phase liquide de la solution contenue dans l'espace interstitiel.

Les étapes c) et d) peuvent être répétées en succession une pluralité de fois.

Avantageusement, le procédé comprend en outre une étape finale de :
f) stérilisation de la microsonde obtenue à l'issue de l'étape e) par passage dans une enceinte remplie d'oxyde d'éthylène.

L'agent pharmacologiquement peut notamment être la dexaméthasone phosphate sodique, la solution étant une solution aqueuse à une concentration comprise entre 0,3 et 5,0 g de dexaméthasone pour 100 ml d'eau désionisée.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 représente l'extrémité distale active, en vue partiellement arrachée, d'une microsonde susceptible de mettre en oeuvre les enseignements de l'invention.
La Figure 2 est une vue agrandie du détail repéré II de la Figure 1.
La Figure 3 est une coupe en section droite, fortement agrandie, de la microsonde des Figures 1 et 2.
La Figure 4 présente les étapes successives du procédé de réalisation de la microsonde selon l'invention.
La Figure 5 est une vue agrandie, en coupe, de l'extrémité distale de la microsonde dans une variante de réalisation de celle-ci.

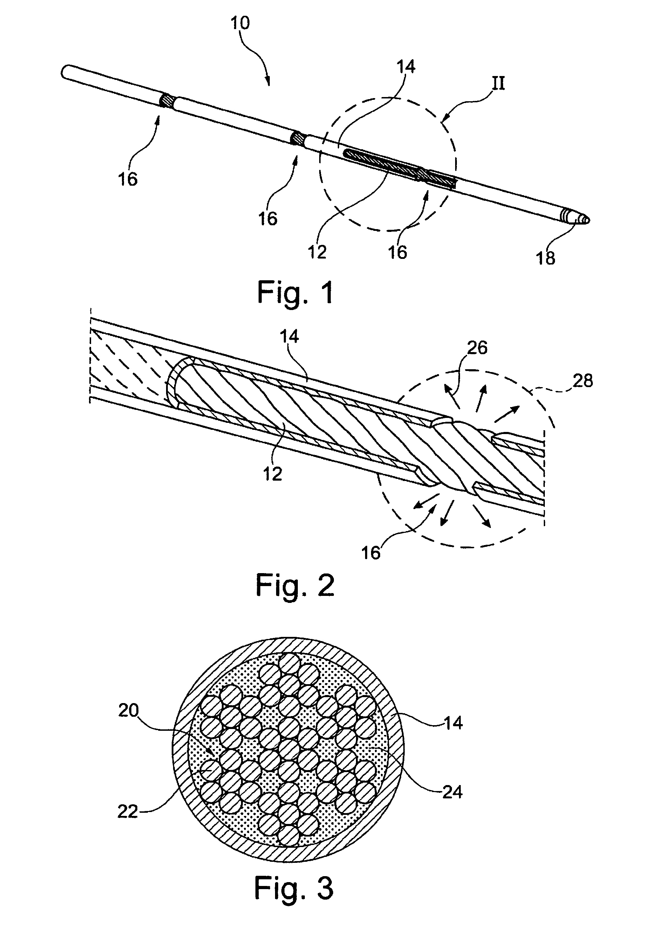

Sur les Figures 1 et 2, on a représenté l'extrémité distale d'une microsonde 10 destinée à être implantée par exemple dans le réseau coronaire. Cette microsonde comprend, dans sa partie d'extrémité distale, active, un câble de coeur 12 gainé par une couche d'isolement 14 en un matériau électriquement isolant, de préférence un matériau de forte inertie chimique tel que le PTFE ou l'ETFE.

Des zones localement dénudées 16 sont ménagées dans la couche d'isolement 14, permettant de mettre à nu le câble de coeur conducteur 12 et former ainsi une électrode correspondante de la sonde. Ces zones 16 sont par exemple dénudées par ablation laser de la couche d'isolement 14. Une électrode d'extrémité ou "tip" 18 peut également être prévue, l'ensemble des éléments 16 et 18 correspondant à une série d'électrodes reliées ensemble en parallèle pour le recueil de potentiels électriques et la délivrance d'impulsions de stimulation. Ceci permet de multiplier les possibilités de contact de la microsonde avec les tissus et d'assurer ainsi une diffusion multizone de l'énergie de stimulation en plusieurs points du ventricule gauche.

La Figure 3 montre plus précisément la structure interne de la microsonde, sous la couche d'isolement 14. Le câble de coeur 12 est en fait constitué par une structure multifilaire torsadée d'une pluralité de microcâbles 20 toronnés ensemble (toronnage que l'on peut voir par exemple sur le détail de la Figure 2), ces microcâbles étant dans l'exemple illustré au nombre de sept. Chaque microcâble 20 est lui-même formé par un toron de plusieurs brins individuels unitaires 22, dans l'exemple illustré au nombre de sept brins avec un brin central entouré de six brins périphériques, de sorte que le câble de coeur 12 est au total constitué d'un ensemble de 7 x 7 = 49 brins. Cette configuration n'est cependant pas limitative, le câble de coeur pouvant être constitué d'un nombre de brins typiquement compris entre 15 et 300 brins, eux-mêmes regroupés en un nombre variable de torons formant les microcâbles.

Diverses structures de ce type sont décrites notamment en détail dans le EP 2 581 107 A1 précité, auquel on pourra se référer pour de plus amples détails, notamment sur le choix des matériaux et la manière dont sont assemblés entre eux les différents brins.

Essentiellement, le câble de coeur 12 est constitué de microcâbles et/ou brins composites alliant un matériau structurant à grande résistance intrinsèque à la fatigue (acier inox, alliage de type MP35NLT, alliage de cobalt, titane, NiTi, etc.) et un matériau radio-opaque (Ta, W, Ir, Pt, Au et leurs alliages). Ces différents fils sont disponibles par exemple auprès de la société Fort Wayne Metals Inc., Fort Wayne, USA.

Le microcâble 12 est revêtu d'une fine couche d'isolement 14, de l'ordre de 25 µm d'épaisseur. Les caractéristiques requises pour cette couche sont : résistance à la fatigue ; isolement électrique ; biocompatibilité à long terme ; biostabilité ; et possibilité de transformation et mise en oeuvre compatible avec le conducteur du câble de coeur. Pour réaliser cette couche d'isolement, on privilégiera les matériaux à forte inertie chimique comme les polymères fluorés, qui présentent également une très bonne qualité d'isolation. Parmi ces composés, on peut citer en particulier l'ETFE (éthylène tétrafluoroéthylène). Les procédés de réalisation de la couche d'isolement sur le câble de coeur sont, par exemple, la co-extrusion sur le conducteur ou l'échauffement d'un tube thermorétractable. Les zones dénudées 16 sont formées par exemple par ablation plasma ou ablation laser de la couche d'ETFE.

Le diamètre extérieur hors tout de la microsonde dans sa partie distale est inférieur à 0,5 mm, par exemple un diamètre extérieur de 0,35 mm. Le diamètre unitaire des brins est généralement compris entre 20 et 40 µm, et l'épaisseur de l'isolant est par exemple de 25 µm d'ETFE.

L'invention tire parti, de façon caractéristique, de la présence d'un volume résiduel constitué par les diverses anfractuosités qui subsistent entre les microcâbles du toron, et entre ces microcâbles 20 et la paroi interne de la couche d'isolement 14.

L'espace interstitiel correspondant 24 constitue physiquement un volume creux non clos, car il est susceptible de communiquer avec l'extérieur à l'endroit des zones dénudées 16.

Dans l'exemple illustré, avec un câble de coeur constitué d'un double toronnage de 7 x 7 = 49 brins et avec les dimensions indiquées de diamètre extérieur et d'épaisseur d'isolant, cet espace interstitiel 24 peut atteindre près de 50 % du volume délimité par la paroi interne de la couche d'isolement 14, soit dans cet exemple un volume de 42,7 mm³ sur une longueur de 1 m pour la partie distale de la microsonde.

On notera que ce volume est constitué uniquement par l'espace interstitiel 24 décrit plus haut, et qu'il ne s'agit pas d'une lumière centrale interne ou autre évidement spécifique formé à l'intérieur de la microsonde. En effet, comme on a pu le comprendre à la lecture de la description plus haut, compte tenu de son très faible diamètre une microsonde ne permet pas, à la différence d'autres types de sondes, l'introduction d'un fil-guide dans une lumière centrale, ni l'utilisation d'une telle lumière par exemple pour l'injection d'un produit de contraste : l'intérieur de la microsonde est au contraire plein et massif (aux espaces interstitiels près).

L'idée de base de l'invention consiste à utiliser l'espace interstitiel 24 pour y loger une charge d'un agent pharmacologiquement actif soluble, qui pourra être progressivement diffusé dans l'environnement de la microsonde via les zones dénudées 16, par lesquelles l'espace interstitiel 24 communique avec le milieu extérieur environnant.

Très avantageusement, cet agent pharmacologiquement actif est un agent anti-inflammatoire, par exemple un glucocorticoïde tel que la dexaméthasone phosphate sodique (ci-après DSP).

Le fait que cet anti-inflammatoire diffuse vers l'extérieur via les zones dénudées 16 (comme illustré en 26 sur la Figure 2) lui permet, précisément, d'agir dans la zone 28 de l'environnement immédiat des électrodes de la microsonde, là où il est précisément nécessaire de réduire l'inflammation pour éviter toute surconsommation par élévation du seuil de capture ou tout risque de perte de capture, comme on l'a expliqué en introduction.

La DSP présente l'avantage d'être un matériau très hygroscopique et hautement soluble dans l'eau, ce qui permet, même avec un volume très réduit, de disposer d'une charge suffisante pour obtenir l'efficacité recherchée pendant la durée requise. Ainsi, avec la valeur indiquée plus haut d'un volume utile résiduel de 42,7 mm³, on peut charger ce volume par exemple avec environ 145 µg de DSP, quantité suffisante et pour autant inférieure à la limite maximale règlementaire de 1 mg. Avec une solution de 2,35 g de DSP pour 100 ml d'eau désionisée, il est toutefois possible d'atteindre la limite de 1 mg de charge initiale (pour un microcâble de 1 mètre) si cela est nécessaire pour augmenter l'efficacité.

Une fois la sonde implantée, celle-ci se trouvera en contact avec le sang du patient et la DSP présente sous forme solide dans l'espace interstitiel 24 se réhydratera aisément par les fenêtres que constituent les zones dénudées 16 du fait de la très grande affinité de cet agent pour l'eau. La DSP se diluera alors dans le sang en sortant progressivement par les zones dénudées 16, réduisant ainsi l'inflammation du tissu adjacent. Le gradient de concentration entre, d'une part, le sang circulant autour de la microsonde et, d'autre part, le milieu intérieur de la microsonde, entretient la diffusion de la DSP vers l'extérieur de la sonde.

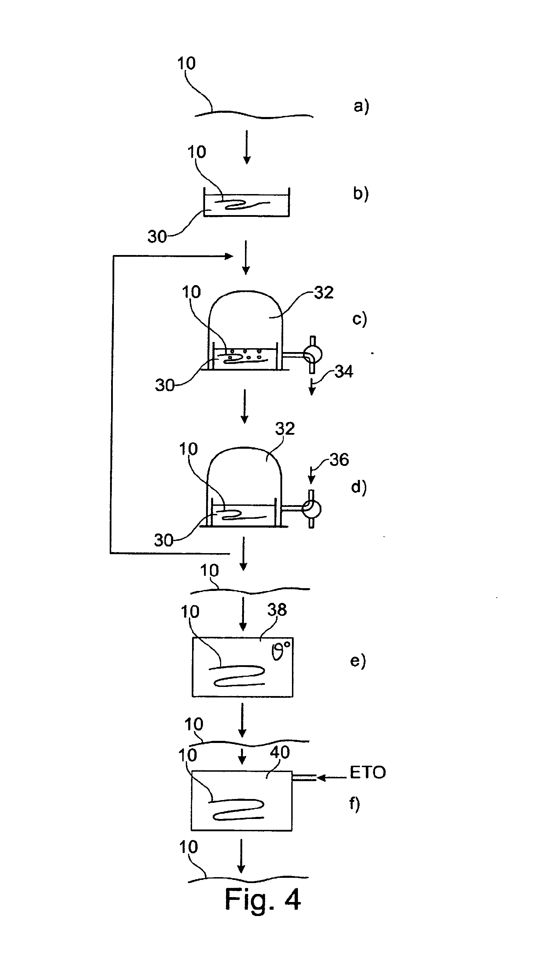

La Figure 4 illustre les étapes successives d'un procédé spécialement adapté à la fabrication d'une sonde telle que celle que l'on vient de décrire. On dispose au départ (étape a) d'une microsonde 10 présentant la structure illustrée Figures 1 à 3, réalisée par l'un des divers procédés exposés par exemple dans le EP 2 581 107 A1 précité, après extrusion de la couche d'isolement 14 et ablation de celle-ci à l'endroit des zones dénudées 16 formant les électrodes.

L'étape suivante (étape b) consiste à immerger cette microsonde 10 dans un bain 30 d'une solution de l'agent pharmacologiquement actif dont on veut charger la sonde, par exemple une solution de 0,3 g de DSP dans 100 ml d'eau désionisée. La sonde est immergée complètement dans cette solution 30 et maintenue dans cette dernière. En variante, une solution organique comme l'alcool peut être également utilisée, pour accélérer l'évaporation à l'étape suivante.

Ensuite (étape c), l'ensemble est placé dans une enceinte étanche 32 reliée à une pompe à vide 34 permettant de vider l'enceinte 32 de l'air initialement présent, jusqu'à stabilisation de la dépression. L'air contenu dans l'espace interstitiel 24 de la sonde est alors évacué par les zones dénudées 16, et remonte à la surface du liquide 30 sous forme de bulles visibles par l'opérateur.

Après stabilisation, la pression dans l'enceinte 32 est rétablie par admission d'air 36 (étape d), ce qui a pour effet de provoquer la pénétration du liquide de la solution 30 dans le corps de la microsonde 10, dans l'espace interstitiel de cette dernière, en remplacement du vide qui y avait été formé à l'étape précédente.

Le cycle des étapes c et d peut être répété plusieurs fois pour améliorer encore l'imprégnation de l'espace interstitiel 24 par la solution de DSP.

Ce cycle c-d (pression atmosphère/vide/pression atmosphérique) peut être éventuellement remplacé ou complété par un cycle de type pression atmosphérique/vide/surpression de quelques atmosphères/pression atmosphérique, ce qui permet d'améliorer le taux de remplissage de l'espace interstitiel 24 au sein de la microsonde (moins de bulles résiduelles) et/ou d'accélérer l'opération de remplissage.

En alternative aux étapes b-d, le remplissage du stéroïde liquide dans l'espace interstitiel de la microsonde peut être réalisé par injection avec une seringue à partir d'une extrémité du microcâble gainé, couplée de manière étanche à la seringue par exemple par un adaptateur de type presse-étoupe, et ceci jusqu'à l'apparition d'une goutte à l'extrémité opposée. Ceci permet un remplissage à une pression supérieure à 1 bar, d'une manière plus complète (moins de bulles résiduelles) et plus rapide. On notera que dans ce cas l'étape de remplissage par le stéroïde peut intervenir soit après gainage du câble mais avant ablation de la couche d'isolement pour former lez zones dénudées (sinon le stéroïde injecté sous pression par une extrémité s'échapperait par ces zones dénudées avant de pouvoir atteindre l'autre extrémité) soit, de préférence, après ablation mais en masquant temporairement les fenêtres des électrodes par un surtube rétracté et retiré après injection.

Dans tous les cas, la sonde 10 est ensuite mise à sécher dans une enceinte 38 dans une atmosphère la plus sèche possible, par exemple pendant 4 jours entre 20 et 40 °C, de manière à extraire le plus complètement la phase liquide de la solution de DSP, phase liquide qui avait servi de véhicule pour la DSP (étape e). Ce séchage provoque une recristallisation de la DSP dans l'espace interstitiel 24 de la microsonde, où la DSP se retrouve alors sous forme d'un matériau sec, soluble.

La sonde peut alors subir un cycle de stérilisation de type conventionnel (étape f), par exemple par passage dans une enceinte 40 alimentée en oxyde d'éthylène ETO.

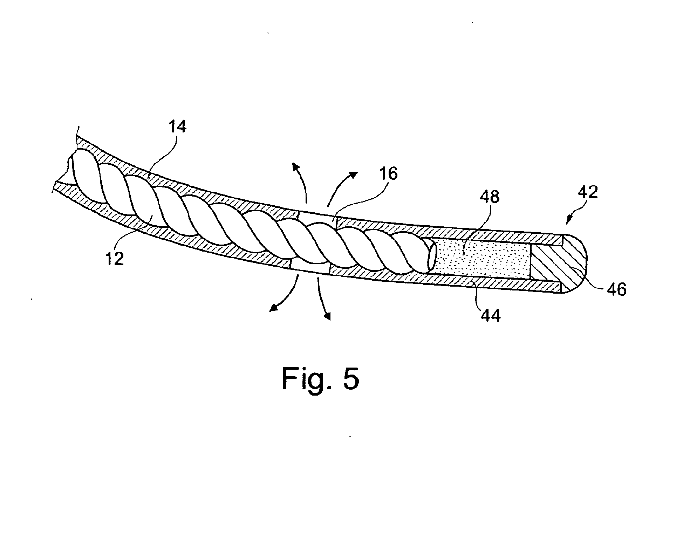

La Figure 5 est une vue agrandie, en coupe, de l'extrémité distale 42 de la microsonde, dans un perfectionnement consistant à prolonger (en 44) la couche d'isolement 14 au-delà de la terminaison du microcâble et à bouchonner (en 46) son extrémité, créant un espace vide 48 qui pourra servir de réservoir au stéroïde (initialement sous forme liquide, puis en poudre après séchage). En particulier, la partie distale atraumatique de la sonde peut assurer cette fonction de réservoir. L'avantage de cette solution tient au fait que les électrodes 16 situées à quelques centimètres de la partie distale 42 de la microsonde disposeront d'autant de réserve de stéroïde à relarguer (réserve contenue dans l'espace 48) que les électrodes proximales, dont les réserves correspondent aux quelques dizaines de centimètres de microsonde menant jusqu'au connecteur de la sonde.

## Revendications

1. Une microsonde (10) de détection/stimulation destinée à être implantée dans des réseaux veineux, artériels ou lymphatiques, comportant une partie distale active dépourvue de lumière centrale et formée par :
- un câble (12) électriquement conducteur formé par un toron de microcâbles (20), chacun des microcâbles étant formé d'un toron de brins métalliques individuels (22) ;
- une couche d'isolement (14) en matériau électriquement isolant, gainant le câble ;
- au moins une zone dénudée (16) ménagée dans la couche d'isolement de manière à former une électrode correspondante de la microsonde ; et
- un agent pharmacologiquement actif, destiné à être progressivement libéré dans l'environnement de la microsonde après implantation de celle-ci,
**caractérisée en ce que** :
- l'agent pharmacologiquement actif est présent sous forme d'un matériau soluble ; et
- l'espace interstitiel (24), délimité par la paroi interne de la couche d'isolement, subsistant entre les brins de chaque microcâble, est rempli par l'agent pharmacologiquement actif.

2. La microsonde de la revendication 1, dans laquelle l'agent pharmacologiquement actif est un agent anti-inflammatoire.

3. La microsonde de la revendication 2, dans laquelle l'agent anti-inflammatoire est un glucocorticoïde.

4. La microsonde de la revendication 3, dans laquelle le glucocorticoïde est la dexaméthasone phosphate sodique.

5. La microsonde de la revendication 2, dans laquelle l'agent anti-inflammatoire est un anti-inflammatoire non stéroïdien.

6. La microsonde de la revendication 1, dans laquelle l'extrémité distale (42) de la microsonde comprend un prolongement (44) de la couche d'isolement (14) au-delà de la terminaison dudit câble (12), ce prolongement définissant un espace vide (48) formant réservoir additionnel pour l'agent pharmacologiquement actif, ce réservoir étant obturé à son extrémité distale par un élément atraumatique (46).

7. Un procédé spécialement adapté à la fabrication d'une microsonde selon l'une de revendications 1 à 6, **caractérisé par** les étapes suivantes :
a) obtention d'une microsonde (10) comportant une partie distale active formée par : un câble (12) électriquement conducteur formé par un toron de microcâbles (20) dont chacun est formé d'un toron de brins métalliques individuels (22), une couche d'isolement (14) en matériau électriquement isolant gainant le câble, et au moins une zone dénudée (16) ménagée dans la couche d'isolement de manière à former une électrode correspondante de la microsonde ;
b) immersion de la microsonde obtenue à l'étape a) dans un bain (30) d'une solution d'un agent pharmacologiquement actif ;
c) placement de la microsonde et du bain de l'étape b) dans une enceinte étanche (32) et mise sous vide de l'enceinte, de manière à évacuer l'air subsistant dans l'espace interstitiel, délimité par la paroi interne de la couche d'isolement, subsistant entre les brins de chaque microcâble de la microsonde ;
d) rétablissement d'une pression, ou établissement d'une surpression, dans l'enceinte, de manière à induire la pénétration de l'agent pharmacologiquement actif dans ledit espace interstitiel ; et
e) extraction de la microsonde hors du bain et séchage (38) de la microsonde, de manière à extraire la phase liquide de la solution contenue dans l'espace interstitiel.

8. Le procédé de la revendication 7, dans lequel les étapes c) et d) sont répétées en succession une pluralité de fois.

9. Le procédé de la revendication 7, comprenant en outre une étape finale de:
f) stérilisation de la microsonde obtenue à l'issue de l'étape e) par passage dans une enceinte (40) remplie d'oxyde d'éthylène.

10. Le procédé de la revendication 7, dans lequel l'agent pharmacologiquement actif est la dexaméthasone phosphate sodique, et la solution (30) est une solution aqueuse à une concentration comprise entre 0,3 et 5,0 g de dexaméthasone pour 100 ml d'eau désionisée.

## Patentansprüche

1. Detektions-/Stimulations-Mikrosonde (10), die dazu bestimmt ist, in venöse, arterielle oder lymphatische Netze implantiert zu werden, und einen aktiven distalen Teil aufweist, der nicht mit einem zentralen Lumen versehen ist und gebildet wird von:
- einem elektrisch leitenden Kabel (12), das von einer Litze aus Mikrokabeln (20) gebildet wird, wobei jedes der Mikrokabel von einer Litze aus einzelnen metallischen Adern (22) gebildet wird;
- einer Isolationsschicht (14) aus elektrisch isolierendem Material, die das Kabel umhüllt;
- wenigstens einem abisolierten Bereich (16), der in der Isolationsschicht ausgebildet ist, um eine entsprechende Elektrode der Mikrosonde zu bilden; und
- einer pharmakologisch aktiven Substanz, die dazu bestimmt ist, in der Umgebung der Mikrosonde nach Implantation derselben allmählich freigesetzt zu werden,
**dadurch gekennzeichnet, dass**:
- die pharmakologisch aktive Substanz in Form eines löslichen Materials vorliegt; und
- der von der Innenwand der Isolationsschicht begrenzte Spaltraum (24), der zwischen den Adern jedes Mikrokabels verbleibt, mit der pharmakologisch aktiven Substanz gefüllt ist.

2. Mikrosonde nach Anspruch 1, wobei die pharmakologisch aktive Substanz eine entzündungshemmende Substanz ist.

3. Mikrosonde nach Anspruch 2, wobei die entzündungshemmende Substanz ein Glukokortikoid ist.

4. Mikrosonde nach Anspruch 3, wobei das Glukokortikoid Natriumdexamethasonphosphat ist.

5. Mikrosonde nach Anspruch 2, wobei die entzündungshemmende Substanz ein nicht-steroidaler Entzündungshemmer ist.

6. Mikrosonde nach Anspruch 1, wobei das distale Ende (42) der Mikrosonde eine Verlängerung (44) der Isolationsschicht (14) über das Ende des Kabels (12) hinaus aufweist, wobei diese Verlängerung einen leeren Raum (48) definiert, der einen zusätzlichen Behälter für die pharmakologisch aktive Substanz bildet, wobei dieser Behälter an seinem distalen Ende durch ein atraumatisches Element (46) verschlossen ist.

7. Verfahren, welches speziell für die Herstellung einer Mikrosonde nach einem der Ansprüche 1 bis 6 geeignet ist, **gekennzeichnet durch** die folgenden Schritte:
a) Herstellen einer Mikrosonde (10), die einen aktiven distalen Teil aufweist, der gebildet wird von: einem elektrisch leitenden Kabel (12), das von einer Litze aus Mikrokabeln (20) gebildet wird, von denen jedes von einer Litze aus einzelnen metallischen Adern (22) gebildet wird, einer Isolationsschicht (14) aus elektrisch isolierendem Material, die das Kabel umhüllt, und wenigstens einem abisolierten Bereich (16), der in der Isolationsschicht ausgebildet ist, um eine entsprechende Elektrode der Mikrosonde zu bilden;
b) Eintauchen der in Schritt a) hergestellten Mikrosonde in ein Bad (30) einer Lösung einer pharmakologisch aktiven Substanz;
c) Anordnen der Mikrosonde und des Bades von Schritt b) in einer dichten Kammer (32) und Evakuieren der Kammer, um die Luft abzusaugen, die in dem von der Innenwand der Isolationsschicht begrenzten Spaltraum verblieben ist, der zwischen den Adern jedes Mikrokabels der Mikrosonde verbleibt;
d) Wiederherstellen eines Drucks oder Herstellen eines Überdrucks in der Kammer, um das Eindringen der pharmakologisch aktiven Substanz in den Spaltraum zu bewirken; und
e) Entnehmen der Mikrosonde aus dem Bad und Trocknen (38) der Mikrosonde, um die flüssige Phase der in dem Spaltraum enthaltenen Lösung zu entfernen.

8. Verfahren nach Anspruch 7, wobei die Schritte c) und d) mehrere Male nacheinander wiederholt werden.

9. Verfahren nach Anspruch 7, welches außerdem folgenden abschließenden Schritt aufweist:
f) Sterilisieren der im Ergebnis von Schritt e) erhaltenen Mikrosonde durch Hindurchbewegen durch eine Kammer (40), die mit Ethylenoxid gefüllt ist.

10. Verfahren nach Anspruch 7, wobei die pharmakologisch aktive Substanz Natriumdexamethasonphosphat ist und die Lösung (30) eine wässrige Lösung mit einer Konzentration ist, die zwischen 0,3 und 5,0 g Dexamethason pro 100 ml deionisiertes Wasser liegt.

## Claims

1. A detection/stimulation micro-lead (10) intended to be implanted in venous, arterial or lymphatic systems, including an active distal part with no central lumen and formed by:
- an electrically conductive cable (12) formed by a strand of micro-cables (20), each of the micro-cables being formed by a strand of individual metal conductors (22);
- an insulating layer (14) made of an electrically insulating material, cladding the cable;
- at least one naked area (16) formed in the insulating layer so as to form a corresponding electrode of the micro-lead; and
- a pharmacologically active agent, intended to be progressively released in the environment of the micro-lead after implantation of the latter,
**characterized in that**:
- the pharmacologically active agent is present as a soluble material; and
- the interstitial space (24), delimited by the internal wall of the insulating layer, existing between the conductors of each micro-cable, is filled with the pharmacologically active agent.

2. The micro-lead of claim 1, wherein the pharmacologically active agent is an anti-inflammatory agent.

3. The micro-lead of claim 2, wherein the anti-inflammatory agent is a glucocorticoid.

4. The micro-lead of claim 3, wherein the glucocorticoid is the dexamethasone sodium phosphate.

5. The micro-lead of claim 2, wherein the anti-inflammatory agent is a non-steroidal anti-inflammatory drug.

6. The micro-lead of claim 1, wherein the distal end (42) of the micro-lead comprises a continuation (44) of the insulating layer (14) beyond the termination of said cable (12), this continuation defining an empty space (48) forming an additional storage for the pharmacologically active agent, said storage being plugged at its distal end by an atraumatic element (46).

7. A method specially adapted to the manufacturing of a micro-lead according to one of claims 1 to 6, **characterized by** the following steps:
a) obtaining a micro-lead (10) including an active distal part formed by: an electrically conductive cable (12) formed by a strand of micro-cables (20), each of which is formed by a strand of individual metal conductors (22), an insulating layer (14) made of an electrically insulating material cladding the cable, and at least one naked area (16) formed in the insulating layer so as to form a corresponding electrode of the micro-lead;
b) immersing the micro-lead obtained at step a) in a bath (30) of a solution of a pharmacologically active agent;
c) placing the micro-lead and the bath of step b) in a sealed enclosure (32) and vacuuming of the enclosure, so as to evacuate the air remaining in the interstitial space, delimited by the internal wall of the insulating layer, existing between the conductors of each micro-cable of the micro-lead;
d) restoring a pressure, or creating an overpressure, in the enclosure, so as to induce the penetration of the pharmacologically active agent into said interstitial space; and
e) extracting the micro-lead from the bath and drying (38) the micro-lead, so as to extract the liquid phase from the solution contained in the interstitial space.

8. The method of claim 7, wherein the steps c) and d) are repeated in succession a plurality of times.

9. The method of claim 7, further comprising a final step of:
f) sterilizing the micro-lead obtained at the end of step e) by passing it in an enclosure (40) filled with ethylene oxide.

10. The method of claim 7, wherein the pharmacologically active agent is the dexamethasone sodium phosphate, and the solution (30) is an aqueous solution in a concentration comprised between 0.3 and 5.0 g of dexamethasone for 100 ml of deionised water.
